# EUROPÄISCHE PATENTSCHRIFT

(11) **EP 3 566 657 B1**
(45) Veröffentlichungstag und Bekanntmachung des Hinweises auf die Patenterteilung: **16.09.2020**
(21) Anmeldenummer: 18171314.0
(22) Anmeldetag: 08.05.2018
(51) Int. Cl.: A61B 17/06, A61B 17/04, B26D 5/24, A61B 17/00, A61B 90/00, B26D 7/10

(54) **VORRICHTUNG UND VERFAHREN ZUM ABLÄNGEN VON CHIRURGISCHEM NAHTMATERIAL**
DEVICE AND METHOD FOR CUTTING SURGICAL SUTURE MATERIAL TO LENGTH
DISPOSITIF ET PROCÉDÉ DE COUPE À LONGUEUR DE MATIÈRE À COUDRE CHIRURGICALE

(43) Veröffentlichungstag der Anmeldung: 13.11.2019
(73) Patentinhaber: Harro Höfliger Verpackungsmaschinen GmbH, 71573 Allmersbach im Tal (DE)
(72) Erfinder: Neff, Ingmar, 71573 Allmersbach im Tal (DE); Weber, Siegfried, 71573 Allmersbach im Tal (DE)
(74) Vertreter: Schmid, Barbara

(56) Entgegenhaltungen:
- EP-A1- 2 245 991
- EP-A2- 0 663 187
- CH-A5- 681 272
- DE-A1- 10 020 602

## Beschreibung

### TECHNISCHES GEBIET

Die Erfindung betrifft eine Vorrichtung und ein Verfahren zum Ablängen von chirurgischem Nahtmaterial, um den Anfang eines Fadens aus chirurgischem Nahtmaterial reproduzierbar zu positionieren und flexibel auf eine definierte Abschneidelänge zu bringen. Mittels einer derartigen Vorrichtung wird somit ein Endlosfaden an chirurgischem Nahtmaterial auf eine gewünschte Länge gekürzt. Das chirurgische Nahtmaterial kann dabei an einer chirurgischen Nadel befestigt werden oder auch als loser Faden vorliegen.

### STAND DER TECHNIK

Vorrichtungen zum Ablängen von chirurgischem Nahtmaterial sind bekannt. Sie besitzen in der Regel eine Fadenrolle mit einem Endlosfaden an chirurgischem Nahtmaterial. Mittels eines linear in einer (horizontalen oder vertikalen) Transportrichtung geführten Fadenvorzugsgreifers wird die gewünschte Länge an chirurgischem Nahtmaterial von der Fadenrolle abgezogen. Dieses Abziehen von der Fadenrolle wird teilweise über einen sogenannten Tänzer gesteuert. Anschließend wird das chirurgische Nahtmaterial an der gewünschten Stelle mittels einer Schneideinheit geschnitten.

In der Regel muss das vordere Fadenende des chirurgischen Nahtmaterials zu einer festen Position vorgezogen werden, da die Verbindung des vorderen Fadenendes mit der chirurgischen Nadel ausschließlich an dieser definierten Position erfolgen kann. Eine Anpassung dieser Fügeposition an unterschiedliche Fadenlängen ist maschinentechnisch nicht möglich. Daher liegt in der Regel immer die maximal mögliche Länge an chirurgischem Nahtmaterial als freie Fadenlänge vor. Die Anpassung der Vorrichtung an unterschiedliche Fadenlängen erfolgt dann durch eine Verschiebung der Schneideinheit in Längsrichtung des freien Fadens. Entsprechend wird auch der Verfahrbereich des Fadenvorzugsgreifers hinsichtlich dessen Startposition angepasst. Abhängig von der Position der Schneideinheit wird das chirurgische Nahtmaterial dann in einem bestimmten Abstand von der Fadenrolle geschnitten. Derartige Schneidmaschinen bauen daher entsprechend lang. Darüber hinaus ist die freie Fadenlänge verhältnismäßig groß, so dass es zu deutlichen Schwingungen im Bereich des abgerollten chirurgischen Nahtmaterials kommen kann. Dies kann sowohl das Einfädeln des Fadens in die Nadel als auch das eigentliche Schneiden des Fadens erschweren.

Aus der CH 681 272 A5 ist eine Vorrichtung zum Ablängen von chirurgischem Nahtmaterial bekannt, bei der das vordere Fadenende zunächst nur eine verhältnismäßig geringe Strecke bis zu der Fügeposition von Nadel und Faden vorgezogen wird. Anschließend kann die Nadel mit dem daran befestigten Faden weiter vorgezogen werden. Mittels Umlenkrollen kann die benötige Länge des chirurgischen Fadens eingestellt werden; sobald diese erreicht ist, kann das chirurgische Nahtmaterial an der gewünschten Stelle geschnitten werden.

### DARSTELLUNG DER ERFINDUNG

Ausgehend von diesem vorbekannten Stand der Technik liegt der Erfindung die Aufgabe zugrunde, eine verbesserte Vorrichtung zum Ablängen von chirurgischem Nahtmaterial auf unterschiedliche Fadenlängen anzugeben, die eine möglichst kompakte Bauweise ermöglicht.

Die erfindungsgemäße Vorrichtung zum Schneiden von chirurgischem Nahtmaterial ist durch die Merkmale des Hauptanspruchs 1 gegeben. Das erfindungsgemäße Verfahren ist durch die Merkmale des nebengeordneten Anspruchs 7 gegeben. Sinnvolle Weiterbildungen der Erfindung sind Gegenstand von sich an diese Ansprüche anschließenden weiteren Ansprüchen.

Die erfindungsgemäße Vorrichtung zum Schneiden von chirurgischem Nahtmaterial besitzt eine Fadenrolle mit einem Endlosfaden des chirurgischen Nahtmaterials sowie einen Fadenvorzugsgreifer zum Abziehen einer definierten Länge an chirurgischem Nahtmaterial. Darüber hinaus ist eine Schneideinheit zum Schneiden des chirurgischen Nahtmaterials vorhanden. Erfindungsgemäß ist eine Umlenkeinrichtung vorgesehen, durch die das chirurgische Nahtmaterial entlang einer sekundären Achse winkelig zur primären Transportrichtung in zumindest eine offene Schlaufe gelegt werden kann. Die Länge dieser offenen Schlaufe kann dabei variabel eingestellt werden. Die Schneideinheit ist dabei zwischen der Umlenkeinrichtung und der Fadenrolle für den Endlosfaden positioniert.

Durch das Vorhandensein der Umlenkeinrichtung ist es nicht länger erforderlich, die Schneideinheit in Längsrichtung verschiebbar auszubilden. Vielmehr ermöglicht es die Umlenkeinrichtung, unterschiedliche Längen an chirurgischem Nahtmaterial abzurollen und abzuschneiden. Die unterschiedlichen Längen werden dabei durch die Länge der offenen Schlaufe bestimmt. Die freie Fadenlänge des chirurgischen Nahtmaterials muss daher auch nicht mehr der maximal möglichen Fadenlänge entsprechend, sondern lediglich noch der minimal erforderlichen Fadenlänge. Auf diese Weise kann die Vorrichtung etwa auf ein Drittel der bisher erforderlichen Länge reduziert werden. Die erfindungsgemäße Vorrichtung kann daher deutlich kürzer und somit auch deutlich kompakter bauen.

Vorzugsweise kann das chirurgische Nahtmaterial durch die Umlenkeinrichtung senkrecht zur primären Transportrichtung in zumindest eine offene Schlaufe gelegt werden. Regelmäßig ist senkrecht zur Längsachse des abgerollten Fadens ausreichend Platz vorhanden, so dass eine solche offene Schlaufe innerhalb der erfindungsgemäßen Vorrichtung ausgebildet werden kann. Die offene Schlaufe kann daher optimal in die Vorrichtung integriert werden, so dass diese kompakt bauen kann.

In einer konstruktiv besonders bevorzugten Ausführungsform kann die Umlenkeinrichtung eine vordere und eine hintere Umlenkeinheit aufweisen, die jeweils stationär ausgebildet sind. Zwischen den beiden stationären Umlenkeinheiten können eine oder mehrere verfahrbare Ausgleichsrollen vorhanden sein. Die zumindest eine Ausgleichsrolle bestimmt dabei durch die Länge ihres Verfahrweges die Schlaufenlänge des chirurgischen Nahtmaterials. Der Verfahrweg der Ausgleichsrolle kann insbesondere an einer Steuereinheit eingegeben werden, so dass bei der erfindungsgemäßen Vorrichtung eine einfache und präzise Einstellung der gewünschten Fadenlänge möglich ist.

Da über die vordere Umlenkeinheit kein Abziehen von chirurgischem Nahtmaterial erfolgt, kann die vordere Umlenkeinheit vorzugsweise als stationäre Fadenumlenkung ausgebildet sein. Dagegen wird über die hintere Umlenkeinheit weiteres chirurgisches Nahtmaterial abgezogen, sobald die Ausgleichsrolle aus ihrer Ausgangsposition verfahren wird. Um ein schonendes Abziehen des chirurgischen Nahtmaterials zu ermöglichen, kann die hintere Umlenkeinheit daher vorzugsweise als stationäre Umlenkrolle ausgebildet sein.

Die Umlenkeinrichtung kann in einer vorteilhaften Ausführungsform höhenverstellbar ausgebildet sein. Da das Abschneiden des chirurgischen Nahtmaterials immer in Transportrichtung gesehen hinter der Umlenkeinrichtung stattfindet, muss der Fadenvorzugsgreifer die Stelle der Umlenkeinrichtung passieren, um das neue vordere Fadenende wieder nach vorne zu transportieren. Ist die Umlenkeinrichtung höhenverstellbar ausgebildet, können die einzelnen Bauteile der Umlenkeinrichtung nach oben oder nach unten ausweichen, um dem Fadenvorzugsgreifer Platz zu machen. Der Fadenvorzugsgreifer kann dadurch insbesondere auf dem Weg zum neuen vorderen Fadenende schneller verfahren werden, darüber hinaus wird dadurch der Faden des chirurgischen Nahtmaterials geschont.

Alternativ oder zusätzlich dazu kann der Fadenvorzugsgreifer beim Zurückfahren ohne Transport des chirurgischen Nahtmaterials und/ oder beim Vorfahren mit Transport des chirurgischen Nahtmaterials einen Bogen um die Umlenkeinrichtung beschreiben. Der Fadenvorzugsgreifer könnte also beispielsweise seitlich oder oberhalb an der Umlenkeinrichtung vorbeifahren.

Bei dem erfindungsgemäßen Verfahren zum Ablängen von chirurgischem Nahtmaterial wird zunächst das chirurgische Nahtmaterial mittels eines Fadenvorzugsgreifers von einer Fadenrolle mit einem Endlosfaden abgezogen. Der Fadenvorzugsgreifer fährt bis zu einer vorbestimmten Endposition vor. Anschließend wird das chirurgische Nahtmaterial durch eine Umlenkeinrichtung in zumindest eine winkelig zur primären Transportrichtung des abgezogenen chirurgischen Nahtmaterials verlaufende offene Schlaufe gelegt. Dadurch wird weiteres chirurgisches Nahtmaterial von der Fadenrolle abgezogen, ohne dass der Fadenvorzugsgreifer weiterfahren würde. Anschließend wird das von der Fadenrolle abgezogene chirurgische Nahtmaterial von einer Schneideinheit abgeschnitten. Die Schneideinheit ist dabei zwischen der Fadenrolle und der Umlenkeinrichtung positioniert.

Vorzugsweise befindet sich die Schneideinheit in der Nähe der Umlenkeinrichtung. Dadurch kann das hintere Fadenende nach dem Durchtrennen des Endlosfadens durch die Umlenkeinrichtung nach unten fallen und bleibt nicht vollständig auf der Umlenkeinrichtung liegen. Dies erleichtert den Weitertransport der abgelängten Fadenstücke.

Durch das erfindungsgemäße Verfahren ist der Verfahrweg des Fadenvorzugsgreifers unabhängig von der Länge des tatsächlich abgeschnittenen chirurgischen Nahtmaterials immer identisch. Der Verfahrweg kann relativ kurz gewählt werden, so dass dieser Verfahrensschritt entsprechend wenig Zeit benötigt. Während weiteres chirurgisches Nahtmaterial durch die Umlenkeinrichtung abgezogen wird, liegt das vordere Fadenende bereits lagefixiert vor, so dass weitere Bearbeitungsschritte an dem vorderen Fadenende vorgenommen werden können. So kann der Fadenvorzugsgreifer beispielsweise bis zu einer Fügestelle zur Verbindung des chirurgischen Nahtmaterials mit einer entsprechenden Nadel fahren. In diesem Fall kann bereits während des weiteren Abziehens des chirurgischen Nahtmaterials eine Fixierung des vorderen Endes des chirurgischen Nahtmaterials in der Nadel erfolgen. Durch die Parallelisierung des Abwickelns des chirurgischen Nahtmaterials zu Arbeitsprozessen am vorderen, positionieren Fadenende hat die Fadenlänge somit nur noch einen minimalen Einfluss auf die eigentliche Bearbeitungszeit. Darüber hinaus steht durch die Parallelisierung auch eine längere Prozesszeit für den eigentlichen Fadentransport durch den Fadenvorzugsgreifer zur Verfügung. Das chirurgische Nahtmaterial kann somit langsamer und damit für das Nahtmaterial schonender durch den Fadenvorzugsgreifer transportiert werden.

Weitere Vorteile und Merkmale der Erfindung sind den in den Ansprüchen ferner angegebenen Merkmalen sowie den nachstehenden Ausführungsbeispielen zu entnehmen.

### KURZE BESCHREIBUNG DER ZEICHNUNG

Die Erfindung wird im Folgenden anhand der in der Zeichnung dargestellten Ausführungsbeispiele näher beschrieben und erläutert. Es zeigen:
- Fig. 1: eine schematische Seitenansicht einer ersten Ausführungsform der Erfindung, bei der sich die Umlenkeinrichtung in ihrer Ausgangsposition befindet,
- Fig. 2: eine schematische Seitenansicht gemäß Fig. 1 während des Verfahrens der Umlenkeinrichtung,
- Fig. 3: eine schematische Seitenansicht gemäß Fig. 2, bei der sich die Umlenkeinrichtung in ihrer Endposition befindet, unmittelbar vor dem Schneiden des chirurgischen Nahtmaterials,
- Fig. 4: eine schematische Seitenansicht der Vorrichtung gemäß Fig. 1 bis 3 mit höhenverstellter Umlenkeinrichtung während des Fadenvorzugs,
- Fig. 5: eine schematische Seitenansicht einer zweiten Ausführungsform der Erfindung, unmittelbar vor dem Schneiden des chirurgischen Nahtmaterials.
- Fig. 6: eine schematische Seitenansicht einer dritten Ausführungsform der Erfindung, bei der sich die Umlenkeinrichtung in ihrer Ausgangsposition befindet,
- Fig. 7: eine schematische Seitenansicht gemäß Fig. 6 während des Verfahrens der Umlenkeinrichtung, und
- Fig. 8: eine schematische Seitenansicht gemäß Fig. 7, bei der sich die Umlenkeinrichtung in ihrer Endposition befindet, unmittelbar vor dem Schneiden des chirurgischen Nahtmaterials.

### WEGE ZUM AUSFÜHREN DER ERFINDUNG

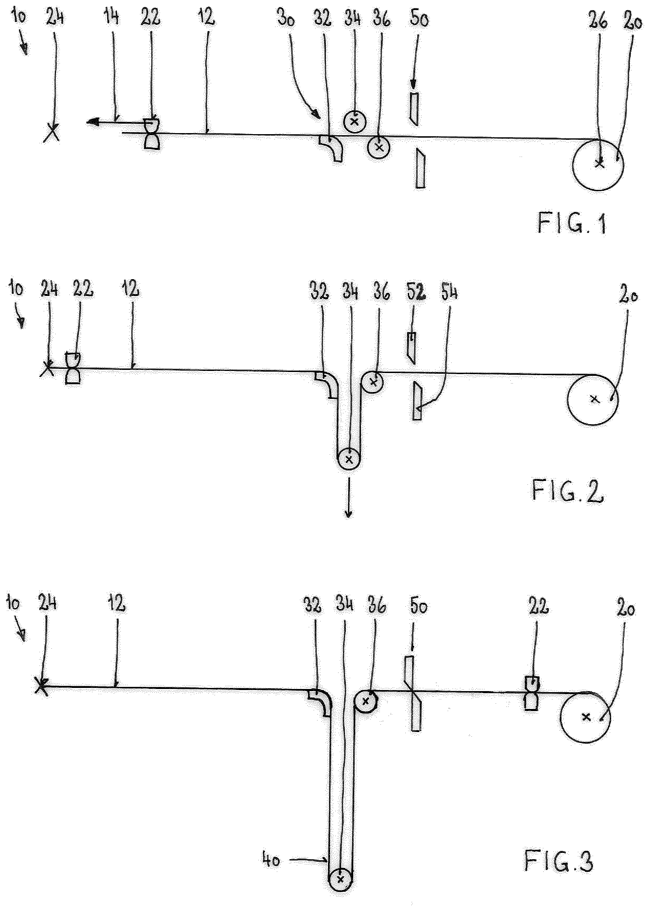

Eine erste Ausführungsform der erfindungsgemäßen Vorrichtung 10 zum Ablängen von chirurgischem Nahtmaterial 12 ist in den Fig. 1 bis 3 schematisch dargestellt. Die Vorrichtung 10 besitzt eine Fadenrolle 20 mit einem Endlosfaden des chirurgischen Nahtmaterials 12. Von dieser Fadenrolle 20 kann das chirurgische Nahtmaterial 12 mittels eines Fadenvorzugsgreifers 22 abgezogen werden. Dazu fährt der Fadenvorzugsgreifer 22 bis zu einer definierten Endposition 24 vor. Die Fadenrolle 20 rotiert dabei um ihre Achse 26 und das chirurgische Nahtmaterial 12 wird im vorliegenden Beispielsfall in horizontaler Richtung (primäre Transportrichtung 14) abgerollt.

Im vorliegenden Beispielsfall findet bei der Endposition 24 eine Verbindung des vorderen Endes des chirurgischen Nahtmaterials 12 mit einer Nadel statt. Dazu wird das vordere Ende des chirurgischen Nahtmaterials 12 ein Stück weit in das hohle Ende der Nadel eingeführt. Anschließend wird das hohle Ende der Nadel zusammengepresst, um das chirurgische Nahtmaterial 12 in der Nadel zu fixieren. Da das vordere Ende des chirurgischen Nahtmaterials 12 nicht mehr bewegt werden muss, nachdem die Endposition 24 erreicht ist, kann der Vorgang der Befestigung des chirurgischen Nahtmaterials 12 in der Nadel unmittelbar nach dem Erreichen der Endposition 24 gestartet werden.

Zwischen der Fadenrolle 20 und der Endposition 24 ist eine Umlenkeinrichtung 30 vorhanden. Die Umlenkeinrichtung 30 besitzt im vorliegenden Ausführungsbeispiel eine stationäre Fadenumlenkung 32. In Vorzugsrichtung hinter der stationären Fadenumlenkung 32 ist eine einzelne verfahrbare Ausgleichsrolle 34 vorhanden. Dahinter ist eine stationäre Umlenkrolle 36 angeordnet. Das chirurgische Nahtmaterial 12 wird oberhalb der stationären Fadenumlenkung 32 und der stationären Umlenkrolle 36 geführt; dagegen befindet sich das chirurgische Nahtmaterial 12 unterhalb der verfahrbaren Ausgleichsrolle 34. Nachdem die Endposition 24 erreicht wurde, kann durch ein Verfahren der Ausgleichsrolle 34 (siehe Fig. 2) weiteres chirurgisches Nahtmaterial 12 von der Fadenrolle 20 abgezogen werden. Dadurch kann ein längeres Stück chirurgischen Nahtmaterials 12 abgeschnitten werden, ohne dass der Fadenvorzugsgreifer 22 weiter verfahren werden müsste.

Durch das Verfahren der Ausgleichsrolle 34 wird das chirurgische Nahtmaterial 12 in eine etwa U-förmige offene Schlaufe 40 gelegt. Die Länge der Schlaufe 40 wird dabei von der Verfahrstrecke der Ausgleichsrolle 34 bestimmt. Diese Verfahrstrecke kann individuell an einer Steuereinheit der Vorrichtung 10 eingegeben werden. Dadurch kann die Gesamtlänge des abgeschnittenen chirurgischen Nahtmaterials 12 auf einfache Weise variiert und an unterschiedliche Bedürfnisse angepasst werden.

Nachdem die Ausgleichsrolle 34 ihre gewünschte Endposition (siehe Fig. 3) erreicht hat, kann das Schneiden des chirurgischen Nahtmaterials 12 erfolgen. Dazu ist zwischen der Umlenkeinrichtung 30 und der Fadenrolle 20 eine Schneideinheit 50 angeordnet. Die Schneideinheit 50 besitzt im vorliegenden Beispielsfall ein oberes Schneidmesser 52 und ein unteres Schneidmesser 54. Die Schneideinheit 50 ist stationär ausgerichtet, da eine Anpassung der Fadenlänge des chirurgischen Nahtmaterials 12 durch die Umlenkeinrichtung 30 erfolgen kann. Dies vereinfacht den konstruktiven Aufbau der Schneideinheit 50 und damit auch der Vorrichtung 10.

Auch alternative Schneideinheiten 50 sind grundsätzlich möglich. So kann die Schneideinheit beispielsweise ein einzelnes Schneidmesser aufweisen, das seitlich des chirurgischen Nahtmaterials 12 angeordnet sein kann. Durch die Spannung im Faden kann ein einzelnes Schneidmesser ausreichen, um einen sauberen und exakten Schnitt zu gewährleisten. Die Bauweise der Schneideinheit ist in diesem Zusammenhang grundsätzlich unerheblich für die Erfindung.

Bevor das chirurgische Nahtmaterial 12 geschnitten wird, muss das hintere Fadenende des chirurgischen Nahtmaterials 12 wieder mittels des Fadenvorzugsgreifers 22 gehalten werden. Dazu wird der Fadenvorzugsgreifer 22 nach dem Fixieren des vorderen Endes des chirurgischen Nahtmaterials 12 in der Nadel wieder in seine Ausgangsposition hinter der Schneideinheit 50 zurückgefahren (siehe Fig. 3). Sofern das vordere Ende des chirurgischen Nahtmaterials 12 nicht in einer Nadel fixiert werden soll, kann auch ein zweiter, hinterer Fadengreifer eingesetzt werden, um das vordere Fadenende des nachfolgenden chirurgischen Nahtmaterials 12 zu fixieren. Dieser hintere Fadengreifer kann stationär unmittelbar hinter der Schneideinheit 50 vorgesehen werden. Nach dem Schneiden des Fadens kann dann der eigentliche Fadenvorzugsgreifer 22 das vordere Fadenende loslassen und zurückfahren, um das nachfolgende vordere Fadenende von dem hinteren Fadengreifer zu übernehmen.

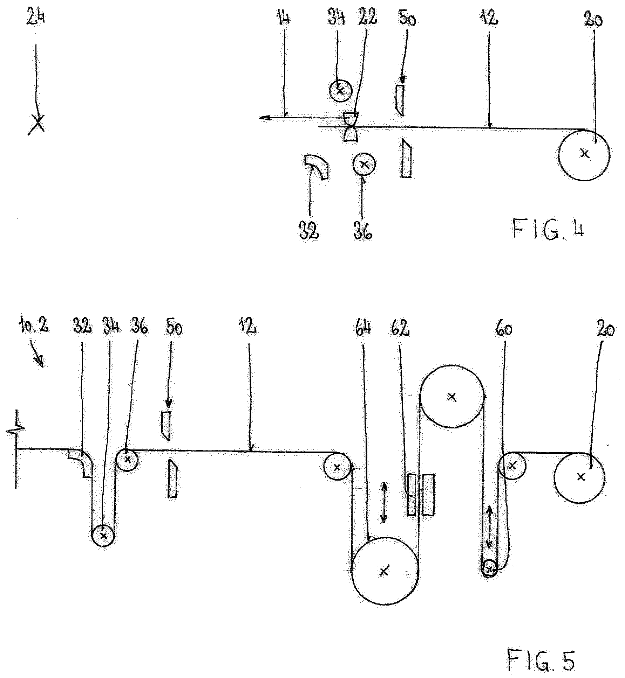

In der Regel erfolgt das Abziehen des chirurgischen Nahtmaterials 12 mittels eines Tänzers 60. Eine zweite Ausführungsform der erfindungsgemäßen Vorrichtung 10.2 mit einem solchen Tänzer 60 zum Abziehen des chirurgischen Nahtmaterials 12 von einer Fadenrolle 20 ist in Fig. 5 schematisch dargestellt. Der Tänzer 60 ist höhenverstellbar ausgebildet und kann bei feststehendem Fadenvorzugsgreifer verfahren werden, um zusätzliches chirurgisches Nahtmaterial 12 von der Fadenrolle 20 abzuziehen. Wird dann der Fadenvorzugsgreifer mit dem vorderen Fadenende verfahren, wird der Tänzer 60 wieder sukzessive in seine Ausgangsposition zurückverfahren, um die entsprechend benötigte Fadenlänge freizugeben. Der Tänzer 60 ist dazu regelmäßig zwischen der Schneideinheit 50 und der Fadenrolle 20 mit dem Endlosfaden angeordnet; im Idealfall befindet sich der Tänzer 60 in unmittelbarer Nähe der Fadenrolle 20.

Um zu verhindern, dass das vordere Fadenende des chirurgischen Nahtmaterials 12 nach dem Schneiden ausfranst, kann das chirurgische Nahtmaterial 12 an der späteren Schnittstelle mittels eines Heizelements 62 verschweißt werden. Das Heizelement 62 ist dabei zwischen dem Tänzer 60 und der Schneideinheit 50 angeordnet. Grundsätzlich wird das chirurgische Nahtmaterial nicht über die gesamte Fadenlänge verschweißt, sondern lediglich an der späteren Schnittstelle. Dazu muss zwischen der Schneideinheit 50 und dem Heizelement 62 ein ganzes Vielfaches der gewünschten Fadenlänge des chirurgischen Nahtmaterials 12 verlaufen. Das Heizelement 62 war daher regelmäßig ebenso wie die Schneideinheit 50 verstellbar ausgebildet. Bei der in Fig. 5 dargestellten Ausführungsform der erfindungsgemäßen Vorrichtung 10.2 ist dagegen zwischen dem Heizelement 62 und der Schneideinheit eine verfahrbare Umlenkrolle 64 ausgebildet, durch die ein Ausgleich der Fadenlänge erfolgen kann. Auf diese Weise kann das Heizelement 62 stationär ausgebildet sein.

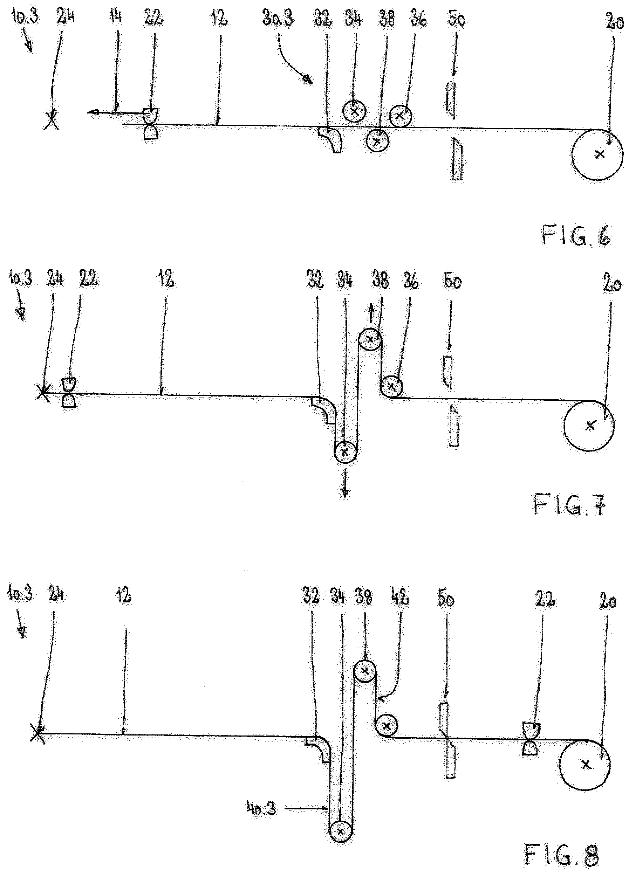

Eine dritte Ausführungsform der erfindungsgemäßen Vorrichtung 10.3 zum Ablängen von chirurgischem Nahtmaterial 12 ist in den Fig. 6 bis 8 schematisch dargestellt. Die Vorrichtung 10.3 besitzt ebenso wie die Vorrichtung 10 eine Fadenrolle 20 mit einem Endlosfaden des chirurgischen Nahtmaterials 12 sowie einen Fadenvorzugsgreifer 22.

Zwischen der Fadenrolle 20 und der Endposition 24 ist eine Umlenkeinrichtung 30.3 vorhanden. Die Umlenkeinrichtung 30.3 besitzt im vorliegenden Ausführungsbeispiel eine stationäre Fadenumlenkung 32.

In Vorzugsrichtung hinter der stationären Fadenumlenkung 32 ist eine erste verfahrbare Ausgleichsrolle 34 sowie eine zweite verfahrbare Ausgleichsrolle 38 vorhanden. Dahinter ist eine stationäre Umlenkrolle 36 angeordnet. Das chirurgische Nahtmaterial 12 wird oberhalb der stationären Fadenumlenkung 32 und der zweiten Ausgleichsrolle 38 geführt; dagegen befindet sich das chirurgische Nahtmaterial 12 unterhalb der ersten verfahrbaren Ausgleichsrolle 34 sowie der stationären Umlenkrolle 36. Nachdem die Endposition 24 erreicht wurde, kann durch ein Verfahren der beiden Ausgleichsrollen 34, 38 (siehe Fig. 7) weiteres chirurgisches Nahtmaterial 12 von der Fadenrolle 20 abgezogen werden. Dadurch kann ein längeres Stück chirurgischen Nahtmaterials 12 abgeschnitten werden, ohne dass der Fadenvorzugsgreifer 22 weiter verfahren werden müsste.

Die beiden Ausgleichsrollen 34, 38 können dabei wie in Fig. 7 und 8 dargestellt gleichzeitig verfahren werden. Alternativ dazu wäre es auch möglich, zunächst lediglich eine der beiden Ausgleichsrollen 34, 38 zu verfahren. Erst wenn der maximale Verfahrweg dieser Ausgleichsrolle 34, 38 erreicht ist, würde mit dem Verfahren der anderen Ausgleichsrolle 34, 38 begonnen werden.

Durch das Verfahren der Ausgleichsrollen 34, 38 wird das chirurgische Nahtmaterial 12 in zwei offene Schlaufen 40.3, 42 gelegt. Die Länge der beiden offenen Schlaufen 40.3, 42 wird dabei von der Verfahrstrecke der entsprechenden Ausgleichsrolle 34, 38 bestimmt. Dadurch kann die Gesamtlänge des abgeschnittenen chirurgischen Nahtmaterials 12 auf einfache Weise variiert und an unterschiedliche Bedürfnisse angepasst werden.

## Patentansprüche

1. Vorrichtung (10, 10.2, 10.3) zum Ablängen von chirurgischem Nahtmaterial (12)
- mit einer Fadenrolle (20) mit einem Endlosfaden des chirurgischen Nahtmaterials (12),
- mit einem Fadenvorzugsgreifer (22) zum Abziehen einer definierten Länge an chirurgischem Nahtmaterial (12), wobei der Fadenvorzugsgreifer (22) bis zu einer definierten Endposition (24) in Form einer Fügestelle zur Verbindung des chirurgischen Nahtmaterials (12) mit einer Nadel verfahrbar ist,
- mit einer Schneideinheit (50) zum Schneiden des chirurgischen Nahtmaterials (12),
- mit einer Umlenkeinrichtung (30, 30.3), durch die das chirurgische Nahtmaterial (12) winkelig zur primären Transportrichtung (14) in zumindest eine offene Schlaufe (40, 40.3, 42) legbar ist, wobei die Länge der offenen Schlaufe (40, 40.3, 42) variabel einstellbar ist,
- wobei die Schneideinheit (50) zwischen der Fadenrolle (20) und der Umlenkeinrichtung (30, 30.3) positioniert ist,
- **dadurch gekennzeichnet, dass**
- die Umlenkeinrichtung (30, 30.3) zwischen der Fadenrolle (20) und der definierten Endposition (24) des Fadenvorzugsgreifers (22) positioniert ist.

2. Vorrichtung nach Anspruch 1,
- **dadurch gekennzeichnet, dass**
- das chirurgische Nahtmaterial (12) durch die Umlenkeinrichtung (30, 30.3) senkrecht zur primären Transportrichtung (14) in zumindest eine offene Schlaufe (40, 40.3, 42) legbar ist.

3. Vorrichtung nach Anspruch 1 oder 2,
- **dadurch gekennzeichnet, dass**
- die Umlenkeinrichtung (30, 30.3) eine vordere und eine hintere stationäre Umlenkeinheit (32, 36) aufweist,
- zwischen den beiden stationären Umlenkeinheiten (32, 36) zumindest eine verfahrbare Ausgleichsrolle (34, 38) vorhanden ist.

4. Vorrichtung nach Anspruch 3,
- **dadurch gekennzeichnet, dass**
- die vordere Umlenkeinheit (32) als stationäre Fadenumlenkung (32) ausgebildet ist.

5. Vorrichtung nach Anspruch 3 oder 4,
- **dadurch gekennzeichnet, dass**
- die hintere Umlenkeinheit (36) als stationäre Umlenkrolle (36) ausgebildet ist.

6. Vorrichtung nach einem der vorstehenden Ansprüche,
- **dadurch gekennzeichnet, dass**
- die Umlenkeinrichtung (30, 30.3) höhenverstellbar ausgebildet ist.

7. Verfahren zum Ablängen von chirurgischem Nahtmaterial (12) mit folgenden Verfahrensschritten,
- ein Fadenvorzugsgreifer (22) zieht das chirurgische Nahtmaterial (12) von einer Fadenrolle (20) mit einem Endlosfaden ab,
- der Fadenvorzugsgreifer (22) fährt bis zu einer vorbestimmten Endposition (24) in Form einer Fügestelle zur Verbindung des chirurgischen Nahtmaterials (12) mit einer Nadel vor,
- anschließend wird das chirurgische Nahtmaterial (12) durch eine zwischen der Fadenrolle (20) und der definierten Endposition (24) des Fadenvorzugsgreifers (22) positionierte Umlenkeinrichtung (30, 30.3) in zumindest eine winkelig zur primären Transportrichtung (14) des abgezogenen chirurgischen Nahtmaterials (12) verlaufende offene Schlaufe (40, 40.3, 42) gelegt,
- anschließend wird das von der Fadenrolle (20) abgerollte chirurgische Nahtmaterial (12) mittels einer Schneideinheit (50) abgeschnitten.

8. Verfahren nach Anspruch 7,
- **dadurch gekennzeichnet, dass**
- während des Verfahrens der Umlenkeinheit (30, 30.3) eine Bearbeitung des vorderen Endes des chirurgischen Nahtmaterials (12), insbesondere eine Fixierung des vorderen Endes des chirurgischen Nahtmaterials (12) in der Nadel, erfolgt.

9. Verfahren nach Anspruch 7 oder 8,
- **dadurch gekennzeichnet, dass**
- die Länge der offenen Schlaufe (40, 40.3, 42) variabel einstellbar ist.

## Claims

1. Device (10, 10.2, 10.3) for cutting-to-length surgical suture material (12),
- having a thread spool (20) having a continuous thread of the surgical suture material (12);
- having a thread-advancing gripper (22) for drawing off a defined length of surgical suture material (12), wherein the thread-advancing gripper (22) is able to be displaced up to a defined terminal position (24) in the form of a joining location for connecting the surgical suture material (12) to a needle;
- having a cutting unit (50) for cutting the surgical suture material (12);
- having a deflection installation (30, 30.3) by way of which the surgical suture material (12) is able to be placed so as to be angled to the primary transport direction (14) in at least one open loop (40, 40.3, 42), wherein the length of the open loop (40. 40.3, 42) is variably adjustable;
- wherein the cutting unit (50) is positioned between the thread spool (20) and the deflection installation (30, 30.3),
- **characterized in that**
- the deflection installation (30, 30.3) is positioned between the thread spool (20) and the defined terminal position (24) of the thread-advancing gripper (22).

2. Device according to Claim 1,
- **characterized in that**
- the surgical suture material (12) by way of the deflection installation (30, 30.3) is able to be placed so as to be perpendicular to the primary transport direction (14) in at least one open loop (40, 40.3, 42).

3. Device according to Claim 1 or 2,
- **characterized in that**
- the deflection installation (30, 30.3) has a front and a rear stationary deflection unit (32, 26) ;
- at least one repositionable compensation roller (34, 38) is present between the two stationary deflection units (32, 36).

4. Device according to Claim 3,
- **characterized in that**
- the front deflection unit (32) is configured as a stationary thread deflection (32).

5. Device according to Claim 3 or 4,
- **characterized in that**
- the rear deflection unit (36) is configured as a stationary deflection roller (36).

6. Device according to one of the preceding claims,
- **characterized in that**
- the deflection installation (30, 30.3) is configured so as to be height-adjustable.

7. Method for cutting-to-length surgical suture material (12), comprising the following method steps:
- a thread-advancing gripper (22) draws off the surgical suture material (12) from a thread spool (20) having a continuous thread;
- the thread advancing gripper (22) moves forward to a predetermined terminal position (24) in the form of a joining location for connecting the surgical suture material (12) to a needle;
- the surgical suture material (12) by way of a deflection installation (30, 30.3) that is positioned between the thread spool (20) and the defined terminal position (24) of the thread-advancing gripper (22) is subsequently placed in at least one open loop (40, 40.3. 42) that runs so as to be angled to the primary transport direction (14) of the drawn-off surgical suture material (12);
- the surgical suture material (12) drawn off from the thread spool (20) is subsequently cut off by means of a cutting unit (50).

8. Method according to Claim 7,
- **characterized in that**
- processing of the leading end of the surgical suture material (12), in particular fixing the leading end of the surgical suture material (12) in the needle, is performed during the repositioning of the deflection unit (30, 30.3).

9. Method according to Claim 7 or 8,
- **characterized in that**
the length of the open loop (40, 40.3, 42) is variably adjustable.

## Revendications

1. Dispositif (10, 10.2, 10.3) pour découper à longueur un matériau de suture chirurgical (12), comprenant
- un rouleau de fil (20) avec un fil sans fin du matériau de suture chirurgical (12),
- un dispositif de préhension d'extraction de fil (22) pour extraire une longueur définie de matériau de suture chirurgical (12), le dispositif de préhension d'extraction de fil (22) pouvant être déplacé jusqu'à une position d'extrémité définie (24) sous la forme d'une zone d'assemblage pour l'assemblage du matériau de suture chirurgical (12) avec une aiguille,
- une unité de coupe (50) pour couper le matériau de suture chirurgical (12),
- un dispositif de renvoi (30, 30.3), par le biais duquel le matériau de suture chirurgical (12) peut être placé suivant un certain angle par rapport à la direction de transport primaire (14) dans au moins une boucle ouverte (40, 40.3, 42), la longueur de la boucle ouverte (40, 40.3, 42) pouvant être ajustée de manière variable,
- dans lequel l'unité de coupe (50) est positionnée entre le rouleau de fil (20) et le dispositif de renvoi (30, 30.3),
**caractérisé en ce que**
- le dispositif de renvoi (30, 30.3) est positionné entre le rouleau de fil (20) et la position d'extrémité définie (24) du dispositif de préhension d'extraction de fil (22).

2. Dispositif selon la revendication 1,
**caractérisé en ce que**
- le matériau de suture chirurgical (12) peut être placé par le dispositif de renvoi (30, 30.3) perpendiculairement à la direction de transport primaire (14) dans au moins une boucle ouverte (40, 40.3, 42).

3. Dispositif selon la revendication 1 ou 2, **caractérisé en ce que**
- le dispositif de renvoi (30, 30.3) présente une unité de renvoi avant et une unité de renvoi arrière stationnaires (32, 36),
- au moins un rouleau d'équilibrage déplaçable (34, 38) est prévu entre les deux unités de renvoi stationnaires (32, 36).

4. Dispositif selon la revendication 3,
**caractérisé en ce que**
- l'unité de renvoi avant (32) est réalisée sous forme de renvoi de fil stationnaire (32).

5. Dispositif selon la revendication 3 ou 4, **caractérisé en ce que**
- l'unité de renvoi arrière (36) est réalisée sous forme de poulie de renvoi stationnaire (36) .

6. Dispositif selon l'une quelconque des revendications précédentes,
**caractérisé en ce que**
- le dispositif de renvoi (30, 30.3) est réalisé de manière réglable en hauteur.

7. Procédé de découpe à longueur de matériau de suture chirurgical (12) comprenant les étapes de procédé suivantes :
- un dispositif de préhension d'extraction de fil (22) tire le matériau de suture chirurgical (12) depuis un rouleau de fil (20) avec un fil sans fin,
- le dispositif de préhension d'extraction de fil (22) avance jusqu'à une position d'extrémité prédéterminée (24) sous la forme d'une zone d'assemblage pour l'assemblage du matériau de suture chirurgical (12) avec une aiguille,
- ensuite le matériau de suture chirurgical (12) est placé par un dispositif de renvoi (30, 30.3) positionné entre le rouleau de fil (20) et la position d'extrémité définie (24) du dispositif de préhension d'extraction de fil (22) dans au moins une boucle ouverte (40, 40.3, 42) s'étendant suivant un angle par rapport à la direction de transport primaire (14) du matériau de suture chirurgical extrait (12),
- ensuite le matériau de suture chirurgical (12) déroulé du rouleau de fil (20) est coupé au moyen d'une unité de coupe (50).

8. Procédé selon la revendication 7,
**caractérisé en ce que**
- pendant le déplacement de l'unité de renvoi (30, 30.3), il se produit un traitement de l'extrémité avant du matériau de suture chirurgical (12), en particulier une fixation de l'extrémité avant du matériau de suture chirurgical (12) dans l'aiguille.

9. Procédé selon la revendication 7 ou 8,
**caractérisé en ce que**
- la longueur de la boucle ouverte (40, 40.3, 42) peut être ajustée de manière variable.
